(19) Europäisches Patentamt
European Patent Office
Office européen des brevets

(11) Publication number: 0 207 901 B1

# (12) EUROPEAN PATENT SPECIFICATION

(45) Date of publication of the patent specification: 28.11.90

(51) Int. Cl.⁵: **C07D 295/14**, C07D 233/61, C07D 339/08, A61K 31/495

(21) Application number: 86830172.2

(22) Date of filing: 19.06.86

(54) **Antianaphylactic and antibronchospastic N-benzhydryldiazacycloalkylalkananilides.**

(30) Priority: 20.06.85 IT 2122585

(43) Date of publication of application: 07.01.87 Bulletin 87/2

(45) Publication of the grant of the patent: 28.11.90 Bulletin 90/48

(84) Designated Contracting States: AT BE CH DE FR GB LI LU NL SE

(56) References cited:
EP-A- 0 032 058
EP-A-01 132 26
EP-A-01 333 23
FR-A- 2 215 235
GB-A- 1 055 100
GB-A- 2 056 968
US-A- 3 244 718
US-A- 3 956 328

**The file contains technical information submitted after the application was filed and not included in this specification**

(73) Proprietor: RECORDATI S.A. CHEMICAL and PHARMACEUTICAL COMPANY, Corso S. Gottardo 54, CH-6830 Chiasso(CH)

(72) Inventor: Nardi, Dante, Via T. Gulli, 47, Milan(IT)
Inventor: Leonardi, Amedeo, Via Poliziano, 16, Milan(IT)
Inventor: Motta, Gianni, Via G. Ungaretti, 10, Barlassina (Milan)(IT)
Inventor: Cazzulani, Pietro, Viale dei Cappuccini, 27, Casal Pusterlengo (Milan)(IT)

(74) Representative: Righetti, Giuseppe, Bugnion S.p.A. Via Carlo Farini, 81, I-20159 Milano(IT)

EP 0 207 901 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

This invention relates to new antianaphylactic and antibronchospastic piperazine derivatives, isomers mixture thereof, individual enantiomers and pharmaceutically acceptable acid addition salts thereof, to a process for their preparation and to pharmaceutical compositions containing them.

In US patent No. 3 244 718, piperazine derivatives are described having a structure similar to that one of the present invention.

Even though, however, it is stated that the phenyl ring attached via a carboxamidoethyl radical to piperazine may be variously substituted, in the preparative Examples only one case is reported wherein said ring is substituted by a trifluoromethyl group, but here the benzhydryl moiety is lacking whereas this moiety is always an integrant part of the compounts of the present invention.

From GB 2 056 968 piperazine derivatives useful in the treatment of allergic symptoms in human being and animals are also known, which however contain neither benzhydryl nor aniline moiety attached to the piperazine ring.

In GB 1 055 100 1,4-disubstituted piperazines and diazepines having antiangiotensine and anthistamine activity are described, in which the benzhydryl moiety is attached to the piperazine ring via a butylene group and wherein the eventual phenyl ring attached to the piperazine ring may be substituted by a lower alkyl, lower alkoxy, halo, nitro or lower alkyl carbonyl group.

It was now surprisingly found that by duly modifying the substituents of the phenyl ring attached via carboxamidoalkyl to piperazine, compounds are obtained showing a high antianaphylactic and antibronchospastic activity.

Object of the present invention are therefore new piperazine derivatives of general formula:

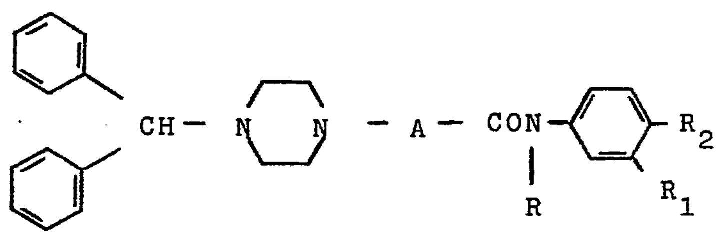

wherein R is hydrogen or lower alkyl, A is a straight or branched lower alkylene, and $R_1$ and $R_2$ are respectively hydrogen, amino, alkylamino, dialkylamino, mono or di(hydroxyalkyl)amino, morpholino, pyrrolidino, piperidino, N-alkylpiperazino, 1,3-dithiolan-2-ylidenamino, N-alkylureido, isomers mixtures thereof, individual enantiomers and pharmaceutically acceptable acid addition salts thereof.

The term lower alkyl and alkyl means here and thereafter a straight or branched alkyl chain containing from 1 to 5 carbon atoms, such as methyl, ethyl, propyl, isopropyl, n-butyl, t-butyl, sec-butyl.

For pharmaceutically acceptable acid addition salts the addition salts are intended with organic or inorganic acids, such as for example hydrochloric, sulfuric, sulfamic, tartaric, hydrobromic acid, hydrogen iodide, glycolic, citric, malic, phosphoric, succinic, acetic, ascorbic acid. They have been prepared by simple adding one or more acid equivalents to the free base.

Intermediates and end-products of the present invention have been prepared by known procedures. In this way the starting compound employed in the following Examples, that is N-benzhydrylpiperazine (II), has been described in several patents and literature or it can be obtained according to known procedures described in literature (see for example: K.E. Hamlin et al., J. Am. Chem. Soc., 71, 2731 (1949); or GB 752 331 and GB 752 332 of July 11 1956).

For preparing the compounds of this invention, the derivative (II) is reacted with an acryloylanilide derivative of formula:

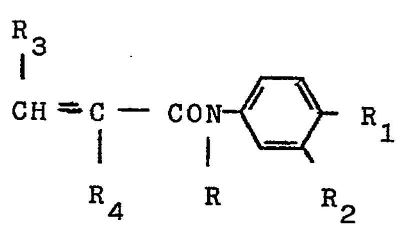

wherein:

$R_3$ and $R_4$ are hydrogen or lower alkyl and R, $R_1$ and $R_2$ have the above meanings, or with a compound of formula:

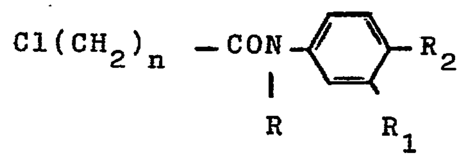

wherein n is an integer comprised between 1 and 5, to give the desired compounds.

Here the reagents are dissolved in equimolar amounts in a suitable solvent, for example dimethylformamide, toluene, xylene, and heated for several hours at a temperature ranging from 50 to 100°C. When as solvent dimethylsulfoxide is employed, the reaction temperature can be greatly lowered, that is one can work also between 10 and 40°C.

At the end of the reaction the desired intermediate thus obtained is collected, for example by filtration or evaporation of the solvent, and eventually purified by silica gel chromatography and/or crystallization from a suitable solvent. When desired, the compound may be eventually transformed into its pharmaceutically acceptable acid addition salt.

The new compounds of the present invention are characterized by a high antianaphylactic and antibronchospastic activity and by a low toxicity.

The $LD_{50}$ of the new piperazine derivatives has been determined in the mouse, both ip and per os, following the method described by C.S. Weil (Biometrics, 8, 249, 1952). The obtained results are reported in the Table.

In order to evaluate the antianaphylactic activity of the new compounds, the method of Goose and Blair was followed, in which passive cutaneous anaphylaxis (PCA) was induced in the rat with homologous antibodies (Immunology, 16, 749, 1969).

Female albino rats were immunized intramuscularly with egg albumin and intraperitoneally with Haemophylus pertussis vaccine. Twelve days after the treatment, the animals were bled and sera thus obtained were injected intradermally into another group of rats. Twenty-four hours later, the animals were challenged with an i.v. solution of ovoalbumin and Evans blue dye, and sacrificed after 30 minutes. The drugs to test were given by different routes of administration and at different times before the challenge.

Inhibition of spots areas ($ED_{50}$) was determined. The obtained results are reported in the Table.

The antibronchospastic activity was performed according to the method of Konzett and Roessler (Arch, exp. Path. Pharmakol., 195, 71, 1940), determining the inhibition of bronchospasm induced in anaesthesized guinea-pig by histamine and acetylcholine. For this purpose, the bronchospasm were induced administering intervenously to the animals 0.5–2.5 µg/kg of histamine or 2.5–10 µg/kg of acetylcholine. Inhibition ($ED_{50}$) was determined one minute after the injection of the drug under test.

Inhibition of passive lung anaphylaxis (PLA) was studied in a model of antigen induced bronchocostriction in sensitized rat, according to the method described by Greenberg et al. (Can. J. Physiol. Pharmacol., 57, 48, 1979). Female albino rats were passively sensitized by intravenous injection of omologous anti-egg albumin serum. Twenty-four hours later the rats were anaesthetized with pentobarbital (35 mg/kg i.p.) and spontaneous respiration was arrested by administration of tubocurarine (0.75 mg/kg i.v.). Bronchocostriction was induced by egg albumin intravenously administered and measured by Konzett and Roessler method (Arch. exp. Path. Pharmakol., 195, 71, 1940). The compounds were injected intravenously one minute before antigen challenge. The inhibitory effect ($ED_{50}$) was evaluated 5 and 10 minutes after i.v. administration of the drugs under test.

The $TXA_2$ inhibition has been evaluated according to the method of Berti et al. (Brit. J. Pharmacol., 68, 467, 1980). Lungs from female or male guinea-pigs were perfused through the pulmonary artery with Krebs bicarbonate (5 ml/min, 37°C). The pulmonary outflow superfused strips of rabbit mesenteric artery and rabbit aorta in cascade, in order to monitor $TXA_2$ activity. These tissues were superfused with a mixture of receptor antagonist and indomethacin to increase their selectivity and sensitivity. Lungs were challenged with a single injection of bradykinin (0.25–0.5 µg) which is known to increase generation of $TXA_2$ from the pulmonary tissue. The new compounds were perfused at different concentrations through the lungs 30 and 60 minutes before agonists.

In the following Table the results obtained with the above tests are reported. The $LD_{50}$ and $DE_{50}$ in PCA test are expressed in mM/kg. The $DE_{50}$ for the antibronchospastic activity (BSP), is expressed in µM/kg i.v. (bronchospasm induced by histamine (H) and acetylcholine (A)).

In $TXA_2$ test test, (C) is the concentration in µg/ml whereas (I%) denotes the maximal inhibition percentage.

In PLA test, the $DE_{50}$ is expressed in µM/kg i.v. (evaluated 5 and 10 minutes after administration).

TABLE

| Compd. | $LD_{50}$ | | $PCA,ED_{50}$ | $BSP,ED_{50}$ | | $TXA_2$ | | $PLA,ED_{50}$ | |
|---|---|---|---|---|---|---|---|---|---|
| No. | ip | os | | H | A | C | I% | 5′ | 10′ |
| (1) | 0.24 | 0.58 | 0.013 | 0.25 | | | | | |
| (2) | 0.26 | 0.34 | >0.069 | | | | | | |
| (3) | | >7 | 0.025 | 1.10 | 0.8 | 5 | 59 | 0.02 | 0.02 |
| (4) | | 5 | 0.008 | 0.21 | 0.4 | 10 | 47 | 0.04 | 0.08 |
| (5) | | 7.2 | 0.028 | 0.23 | | | | | |
| (6) | >2.33 | >7 | 0.010 | 0.12 | 0.4 | 10 | 0.8 | 0.65 | 0.69 |
| (7) | 1.74 | >5.90 | 0.073 | | | | | | |
| (8) | 0.23 | 0.43 | 0.010 | 1.10 | >3 | | | 0.84 | 0.84 |

The following examples illustrate the invention.

PREPARATION OF THE INTERMEDIATES

A mixture of 12.6 g of N,N-dimethyl-m-phenylendiamine dihydrochloride, 4.8 g of sodium hydroxide and 60 ml of acetic was stirred for 5 hours at 20–25°C. At the same temperature, 5.76 ml of acryloylchloride were added dropwise. The suspension was stirred and, one hour later, a solution of 36.3 g of sodium acetatetrihydrate in 90 ml of water was slowly added by dropping. The solution was allowed ot stand for 24 hours, then it was diluted with 150 ml of water and alkalinized with concentrated sodium hydroxide. The oily precipitate was extracted with ethyl acetate and the organic phase dried on anhydrous sodium sulphate. The residue obtained by evaporation of the solvent was purified by silica gel chromatography eluting with ethyl acetate. The fractions unitary at TLC were collected and the solvent was evaporated off. The residue was crystallized from ethyl acetate/petroleum ether to give 5.8 g of 3′-dimethylaminoacryloylanilide melting at 101–103°C.

Operating as described above the following intermediates habe been obtained:
4′-diethylaminoacryloylanilide, mp 122°C;
4′-dimethylaminoacryloylanilide, mp 143–144°C;
4′-(1-piperidinyl)acryloylanilide, mp 147–148°C;
4′-(1-morpholinyl)acryloylanilide, mp 220–222°C;
4′-dimethylamino-N-methylacryloylanilide, mp 59–62°C;
4′-(4-methyl-1-piperazinyl)acryloylanilide, mp 191–193°C;
4′-(1-imidazolyl)acryloylanilide, mp 181–183°C;
4′-bis-(2-hydroxyethyl)aminoacryloylanilide, mp 142.5°C;
3′-nitro-N-methylacryloylanilide, mp 68°C.

Operating as described above but employing a duly substituted acid chloride instead of acryloylchloride, the following compounds were prepared:
4′-dimethylaminocrotonylanilide, mp 173–174°C;
4′-dimethylaminomethacryloylanilide, mp 127–139°C;

b) a mixture of 21 g of N,N-dimethyl-p-phenylendiamine dihydrochloride, 41.8 ml of $Et_3N$ and 80 ml of chloroform was stirred at 20–23°C for 30 minutes. At the same temperature and within twenty minutes, a solution of 11.3 ml of 4-chlorobutyrilchloride in 20 ml of chloroform was added dropwise. The mixture was stirred for two hours and then diluted and shaked with about 300 ml of water. The organic phase was separated off, dried on calcium chloride/anhydrous sodium sulphate, filtered and evaporated to dryness. The residue thus obtained was purified by silica gel chromatography employing ethyl acetate as eluent. The fractions unitary to TLC were collected, the solvent was evaporated off under vacuum and the residue crystallized from carbon tetrachloride to give 14.8 g of
4′-dimethylamino-4-chlorobutyrylanilide melting at 110°C.

c) Operating as decribed in the following Example 1, but employing 3′-nitroacryloylanilide instead of 4′-diethylaminoacryloylanilide, was obtained the 3-(4-benzhydryl-1-piperazinyl)-N,3-nitrophenylpropionamide intermediate melting at 158–159°C.

In the same manner employing 4′-nitroacryloylanilide was obtained the corresponding N,4-nitrophenylpropionamide derivative melting at 177–179°C.

EXAMPLE 1

3-(4-benzhydryl-1-piperazinyl)-N,4-diethylaminophenylpropionamide (9)

A mixture comprising 8.82 g of N-benzhydrylpiperazine, 7.64 g of 4′-diethylaminoacryloylanilide and 70 ml of toluene was refluxed for 6 hours under stirring. At the end of the reaction, the solvent was

evaporated under vacuum and the crude product was chromatographed on silica gel column using ethyl acetate as eluent. The fractions containing the desired product were collected, the solvent was evaporated off and the crude product was crystallized from ethyl acetate and petroleum ether to give 4.8 g of the title compound melting at 112–114°C.

Operating as described above but employing a duly substituted acryloylanilide instead of 4′-diethylaminoacryloylanilide, the following compounds were prepared:
3-(4-benzhydryl-1-piperazinyl)-N,4-dimethylaminophenylpropionamide (3), mp 155–157°C;
3-(4-benzhydryl-1-piperazinyl)-N,4-(1-morpholino)phenylpropionamide (10), mp 157–159°C;
3-(4-benzhydryl-1-piperazinyl)-N,4-(1-piperidino)phenylpropionamide (11), mp 135–137°C;
3-(4-benzhydryl-1-piperazinyl)-N-methyl-N,4-dimethylaminophenylpropionamide (12), mp 139–141°C;
3-(4-benzhydryl-1-piperazinyl)-N-(4-bis-(2-hydroxyethyl)aminophenyl)propionamide (13), mp 150–152°C.

Operating in the same manner, but employing 4′-dimethylaminocrotonylanilide instead of 4′-diethylaminoacryloylanilide, and using xilene instead of toluene as solvent,
3-(4-benzhydryl-1-piperazinyl)-N,4-dimethylaminophenylbutyramide (14), mp 148°C has been obtained.

EXAMPLE 2

3-(4-Benzhydryl-1-piperazinyl)-N,4-(4-methyl-1-piperazinyl)phenylpropionamide (15)

A mixture comprising 2.45 g of 4′-(4-methyl-1-piperazinyl)acryloylanilide, 2.52 g of N-benzhydrylpiperazine and 20 ml of DMSO was stirred for 6 hours at 80°C. At the end of the reaction the cooled solution was poured in about 200 ml of water and the mixture was extracted with ethyl acetate. The organic phase was separated, washed with water, dried on anhydrous sodium sulphate, filtered and evaporated to dryness. The residue thus obtained was purified on silica gel column, eluting with a 5% solution of acetic acid in water. The fractions containing the desired product were collected and alkalinized adding 30% sodium hydroxide. The precipitate was collected by filtration dried and crystallized from ethyl acetate/petroleum ether to give 2 g of the title compound melting at 156–158°C.

Operating as described above, but employing 4′-(1-imidazolyl)acryloylanilide instead of 4′-(4-methyl-1-piperazinyl)acryloylanilide, was obtained the 3-(4-benzhydryl-1-piperazinyl)-N,4-(1-imidazolyl) phenylpropionamide (16), mp 103–105°C (dec).

EXAMPLE 3

3-(4-Benzhydryl-1-piperazinyl)-N,3-aminophenylpropionamide (4)

A mixture comprising 12 g of powder zinc, 8.38 g of calcium chloride dihydrate in 12 ml of water and 39 ml of ethanol was stirred and heated for one hour at 80°C, 13.32 g of 3-(4-benzhydryl-1-piperazinyl)-N,3-nitrophenylpropionamide, prepared as described in Example 1, were then added and the mixture was stirred for further 5 hours at the same temperature. At the end of the reaction the insoluble was filtered off and the solvent was evaporated under vacuum. The residue thus obtained was purified by column chromatography (silica gel) with chloroform/methanol 95:5 as eluent. Fractions unitary at TLC were collected, the solvent was evaporated under vacuum and the residue crystallized from ethanol (or ethyl acetate) and petroleum ether to give 5.9 g of the title product melting at 151–154°C.

Operating as described above, but starting from 3-(4-benzhydryl-1-piperazinyl)-N,4-nitrophenylpropionamide, the corresponding 4-aminoderivative (5) melting at 170–172°C was obtained.

EXAMPLE 4

3-(4-Benzhydryl-1-piperazinyl)-N,4-methylureidophenylpropionamide (7)

A mixture comprising 6.21 g of 3-(4-benzhydryl-1-piperazinyl)-N,4-aminophenylpropionamide, prepared as described in the previous Example, 0.87 g of methylisocyanate and 30 ml of toluene was heated under stirring for 3 hours at 100°C. At the end of the reaction the mixture was cooled and the solid thus precipitated was collected by filtration and crystallized from acetone to give 6.5 g of the title compound melting at 205–207°C. The hydrochloride, obtained by adding to the base hydrochloric acid in ethanol, melted at 160–163°C.

EXAMPLE 5

3-(4-Benzhydryl-1-piperazinyl)-N,4-dimethylaminophenylbutyramide (8)

A mixture of 10.83 g of 4′-dimethylamino-4-chlorobutyrylanilide, 22.68 g of N-benzhydrylpiperazine, 7.38 g of potassium iodide and 180 ml of DMSO was stirred for 7 days at 18–23°C. At the end of the reaction the mixture was poured in about 1.8 l of 0.25 N sodium hydroxide and extracted with ethyl acetate. The

organic phase thus separated was dried on anhydrous sodium sulphate, filtered and evaporated to dryness. The residue thus obtained was purified on silica gel column eluting with ethyl acetate.

The fractions containing the desired product were collected, the solvent was evaporated off and the residue was dissolved in ethanol and added with hydrochloric acid in ethanol and subsequently with ethyl ether until turbidity. The hydrochloride thus crystallized was collected by filtration and recrystallized from ethanol or methanol to give 8 g of the title compound melting at 192–195°C (dec).

EXAMPLE 6

3-(4-Benzhydryl-1-piperazinyl)-N,3-methylaminophenylpropionamide (1)

A mixture comprising 15.5 g of the compound (4) described in Example 3, 4.4 g of succinimide, 3.37 g of 37% formaldehyde solution and 92 ml of ethanol was refluxed under stirring for 10 hours. After cooling at 20–25°C the solvent was evaporated off under vacuum and the residue was purified on silica gel column using ethyl ether/methanol 9:1 as eluent. From the last fractions, which were collected and evaporated to dryness, 10.5 g of 3-(4-benzhydryl-1-piperazinyl)-N,3-(succinimidoethyl)aminophenylpropionamide as dark oil were obtained, which was then employed as such for the next reaction. To 10.5 g of the product obtained as described above, dissolved in 30 ml of DMSO at 50–60°C, 0.88 g of sodium borohydride were added dropwise. When the adding was over, the solution thus obtained was heated under stirring for 30 minutes at 100°C and subsequently cooled at 20–25°C, poured in about 400 ml of water, acidified with diluted hydrochloric acid and, after standing overnight, alkalinized with diluted sodium hydroxide.

The precipitate thus obtained was collected by filtration and purified by silica gel chromatography employing ethyl acetate as eluent. The fractions unitary at TLC were collected, the solvent was evaporated off, the residue dissolved in ethanol and the solution acidified with hydrochloric acid in ethanol. Crystallized a solid product, that was collected by filtration and recrystallized from methanol to give 4.5 of the title compound as trihydrochloride, emihydrate, melting at 203–204°C.

Operating as described above, but employing
3-(4-benzhydryl-1-piperazinyl)-N,4-aminophenylpropionamide (5) instead of 3-aminophenylderivative (4), was obtained the
3-(4-benzhydryl-1-piperazinyl)-N,4-methylaminophenylpropionamide (17), melting at 140–141°C.

EXAMPLE 7

3-(4-Benzhydryl-1-piperazinyl)-N,3-aminophenyl-N-methylpropionamide (6)

A mixture of 19 g of 3'-nitro-N-methylacryloylanilide, 23 g of N-benzhydrylpiperazine and 185 ml of toluene was refluxed under stirring for 3 hours. At the end of the reaction the solvent was evaporated off under vacuum and the residue was dissolved in ethyl acetate and filtered on porous set containing silica gel in order to eliminate the N-benzhydrylpiperazine still present.

The filtrate was then evaporated to dryness, the residue dissolved in ethyl ether and then filtered. By adding hydrochloric acid in ethanol, was collected the 3-(4-benzhydryl-1-piperazinyl-N-methyl-N,3-nitrophenylpropionamide hydrochloride emihydrate, which was then dissolved in ethanol/ethyl acetate and crystallized until constant melting point was reached (180–182°C) giving a yield of 17.2 g.

A mixture comprising 15.6 g of the compound described above, 35.5 g of stannous chloride dihydrate and 95 ml of ethanol was heated under stirring for 3 hours at 70°C in nitrogen atmosphere. At the end of the reaction the mixture was cooled at 20–25°C, the solvent was evaporated off under vacuum and the residue was treated with about 400 ml of water. After having alkalinized with diluted sodium hydroxide, an extraction with ethyl acetate took place. The solvent was evaporated, the residue was milled with about 150 ml of water and the solid thus obtained collected by filtration, air dried and crystallized from ethanol or isopropyl acetate to give 10.2 g of the title compound melting at 144–146°C.

EXAMPLE 8

3-(4-Benzhydryl-1-piperazinyl)-N,3-(1,3-dithiolan-2-yliden)aminophenylpropionamide (18)

To 1.11 g of (1,3-dithiolan-2-yliden)methylsulfonium iodide (prepared as described by R. Mayer and K. Schafer, J. Prakt. Chem., 26, 279 (1964)) dissolved in 3.3 ml of DMF at 20–23°C, were added dropwise within 20 minutes, 1.66 g of 3-(4-benzhydryl-1-piperazinyl)-N,3-nitrophenyl propionamide dissolved in 13.3 ml of DMF.

The mixture was heated under stirring for 13 hours at 50°C, then it was cooled and poured in 100 ml of 0.2 N sodium hydroxide. The crude product was collected by filtration and purified by chromatography on silica gel column employing chloroform/methanol 95:5 as eluent. The fractions unitary at TLC were collected, the solvent was evaporated off and the resulting residue was crystallized from $CH_3CN$ to give 0.3 g of the title compound melting at 219–220°C.

EXAMPLE 9

2-Methyl-3-(4-benzhydryl-1-piperazinyl)-N,4-dimethylaminophenylpropionamide (19)

A mixture of 12.6 g of 4'-dimethylaminomethacryloylanilide, 16.63 g of N-benzhydrylpiperazine and 43 ml of N,N-dimethylacetamide was stirred and refluxed for 3 hours. At the end of the reaction the mixture was cooled, poured in about 600 ml of water, extracted with ethyl ether and washed with water. The phases were separated and the organic layer was dried on anhydrous sodium sulphate. The solvent was evaporated and the residue thus obtained was filtered off and purified on a silica gel column employing a 3:2 ethyl acetate/petroleum ether mixture and then only ethyl acetate as eluent. The fractions under vacuum, the residue was dissolved in ethanol and acidified with hydrochloric acid in ethanol. The title compound was collected by filtration as trihydrochloride and crystallized from methanol and ethyl ether until mp of 228–232°C (yield 9.5 g).

**Claims**

1. A compound of formula:

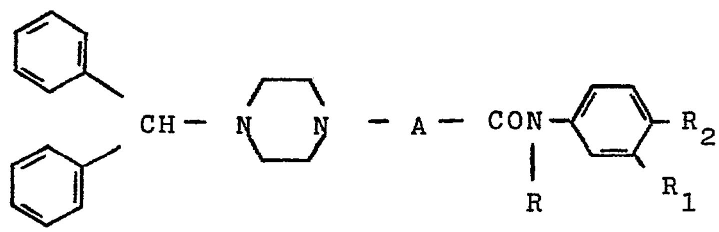

wherein:
R is hydrogen or alkyl having from 1 to 5 carbon atoms,
A is a straight or branched alkylene chain having from 1 to 5 carbon atoms, and
$R_1$ and $R_2$ are each hydrogen, amino, alkylamino, dialkylamino, mono and di(hydroxyalkyl)amino containing from 1 to 5 carbon atoms in the alkyl group, morpholino, piperidino, N–$C_1$–$C_5$-alkylpiperazino, 1,3-dithiolan-2-ylidenamino, N–$C_1$–$C_5$-alkylureido, their isomers mixtures and individual enantiomers, as well as the pharmacologically acceptable acid addition salts thereof.

2. A compound of formula (I) according to claim 1 selected among:
3-(4-benzhydryl-1-piperazinyl)-N,4-diethylaminophenylpropionamide,
3-(4-benzhydryl-1-piperazinyl)-N,4-(1-morpholino)phenylpropionamide,
3-(4-benzhydryl-1-piperazinyl)-N,4-(1-piperidino)phenylpropionamide,
3-(4-benzhydryl-1-piperazinyl)-N,3-dimethylaminophenylpropionamide,
3-(4-benzhydryl-1-piperazinyl)-N,4-dimethylaminophenylpropionamide,
3-(4-benzhydryl-1-piperazinyl)-N-methyl-N,4-dimethylaminophenylpropionamide,
3-(4-benzhydryl-1-piperazinyl)-N-(4,bis-(2-hydroxyethyl)aminophenyl)propionamide,
3-(4-benzhydryl-1-piperazinyl)-N,4-dimethylaminophenylbutyramide,
3-(4-benzhydryl-1-piperazinyl)-N,4-(4-methyl-1-piperazinyl)phenylpropionamide,
3-(4-benzhydryl-1-piperazinyl)-N,4-(1-imidazolyl)phenylpropionamide,
3-(4-benzhydryl-1-piperazinyl)-N,3-aminophenylpropionamide,
3-(4-benzhydryl-1-piperazinyl)-N-4-aminophenylpropionamide,
3-(4-benzhydryl-1-piperazinyl)-N,4-methylureidophenylpropionamide,
4-(4-benzhydryl-1-piperazinyl)-N,4-dimethylaminophenylbutyramide,
3-(4-benzhydryl-1-piperazinyl)-N,3-methylaminophenylpropionamide,
3-(4-benzhydryl-1-piperazinyl)-N,4-methylaminophenylpropionamide,
3-(4-benzhydryl-1-piperazinyl)-N,3-aminophenyl-N-methylpropionamide,
3-(4-benzhydryl-1-piperazinyl)-N,3-(1,3-dithiolan-2-yliden)aminophenylpropionamide,
2-methyl-3-(4-benzhydryl-1-piperazinyl)-N,4-dimethylaminophenylpropionamide.

3. A process for preparing a compound of formula (I) according to claim 1, characterized in that N-benzhydrylpiperazine (II) is reacted with an acryloylanilide derivative of formula:

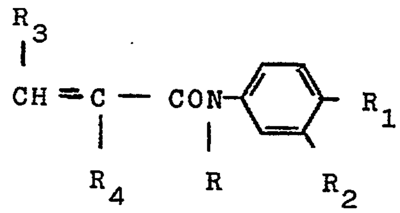

wherein:
R, $R_1$ and $R_2$ have the meaning stated in claim 1 and $R_3$ and $R_4$ are hydrogen or $C_1$–$C_5$-alkyl or with a compound of formula:

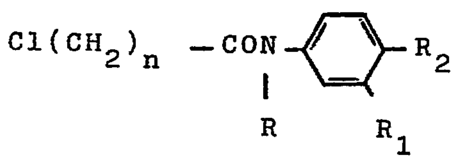

wherein:
n is a number comprised between 1 and 5, in a suitable solvent, for several hours, at a temperature comprised between 50 and 100°C and the product thus obtained is isolated and purified according to known procedures.

4. A process according to claim 3, characterized in that the solvent is selected among dimethylformamide, toluene or xilene.

5. Pharmaceutical compositions having antianaphylactic and antibronchospastic activity and containing a compound of claim 1 and 2.

**Patentansprüche**

1. Eine Verbindung mit Formel:

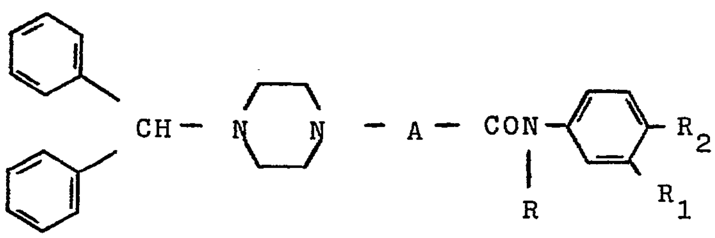

worin:
R Wasserstoff oder Alkyl mit 1 bis 5 Kohlenstoffatomen ist,
A eine gerade oder verzweigte Alkylenkette mit 1 bis 5 Kohlenstoffatomen ist, und
$R_1$ und $R_2$ jeweils Wasserstoff, Amino, Alkylamino, Dialkylamino, Mono- und Di-(Hydroxyalkyl)Amino mit 1 bis 5 Kohlenstoffatomen in der Alkylgruppe, Morpholin, Piperidin, N–$C_1$–$V_5$-Alkylpiperazin, 1,3-Dithiolan-2-Ylidenamin, N–$C_1$–$C_5$-Alkylureid, deren isomere Mischungen und individuellen Enantiomere, sowie die pharmakologisch akzeptablen Säureadditionssalze derselben sind.

2. Eine Verbindung der Formel (I) nach Anspruch 1, ausgewählt unter:
3-(4-Benzhydryl-1-Piperazinyl)-N,4-Diäthylaminophenyl-Propionamid,
3-(4-Benzhydryl-1-Piperazinyl)-N,4-(1-Morpholin) Phenylpropionamid,
3-(4-Benzhydryl-1-Piperazinyl)-N,4-(1-Piperidin) Phenylpropionamid,
3-(4-Benzhydryl-1-Piperazinyl)-N,3-Dimethylaminophenyl-Propionamid,
3-(4-Benzhydryl-1-Piperazinyl)-N,4-Dimethylaminophenyl-Propionamid,
3-(4-Benzhydryl-1-Piperazinyl)-N-Methyl-N,4-Dimethyl-Aminophenylpropionamid,
3-(4-Benzhydryl-1-Piperazinyl)-N-(4,bis-(2-Hydroxy-Äthyl) Aminophenyl) Propionamid,
3-(4-Benzhydryl-1-Piperazinyl)-N,4-Dimethylamino-Phenylbutyramide,
3-(4-Benzhydryl-1-Piperazinyl)-N,4-(4-Methyl-1-Piperazinyl) Phenylpropionamid,
3-(4-Benzhydryl-1-Piperazinyl)-N,4-(1-Imidazolyl) Phenylpropionamid,
3-(4-Benzhydryl-1-Piperazinyl)-N,3-Aminophenyl-Propionamid,
3-(4-Benzhydryl-1-Piperazinyl)-N,4-Aminophenyl-Propionamid,
3-(4-Benzhydryl-1-Piperazinyl)-N,4-Methylureidphenyl-Propionamid,

4-(4-Benzhydryl-1-Piperazinyl)-N,4-Dimethylamino-Phenylbutyramid,
3-(4-Benzhydryl-1-Piperazinyl)-N,3-Methylamino-Phenylpropionamid,
3-(4-Benzhydryl-1-Piperazinyl)-N,4-Methylamino-Phenylpropionamid,
3-(4-Benzhydryl-1-Piperazinyl)-N,3-Aminophenyl-N-Methylpropionamid,
3-(4-Benzhydryl-1-Piperazinyl)-N,4-(1,3-Dithiolan-2-Yliden) Aminophenylpropionamid,
2-Methyl-3-(4-Benzhydryl-1-Piperazinyl)-N,4-Dimethyl-Aminophenylpropionamid.

3. Ein Verfahren zur Zubereitung einer Verbindung der Formel (I) nach Anspruch 1, dadurch gekennzeichnet, daß N-Benzhydrylpiperazine (II) mit einem Acryloylanilid-Derivat der Formel:

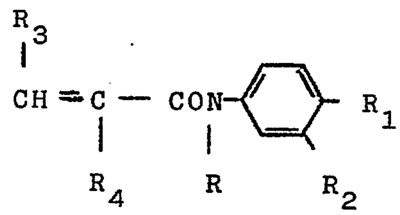

umgesetzt wird, worin:
R, $R_1$ und $R_2$ die in Anspruch 1 festgesetzten Bedeutungen besitzen und $R_3$ und $R_4$ Wasserstoff oder $C_1$–$C_5$ Alkyl sind oder mit einer Verbindung der Formel:

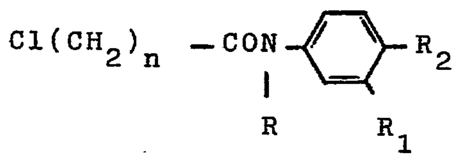

worin:
n eine Zahl zwischen 1 und 5 ist,
in einem geeignetem Lösungsmittel für einige Stunden bei einer Temperatur zwischen 50 und 100°C umgesetzt wird und das dementsprechend erhaltene Produkt isoliert und gemäß den bekannten Verfahren gereinigt wird.

4. Ein Verfahren nach Anspruch 3, dadurch gekennzeichnet, daß das Lösungsmittel zwischen Dimethylformamid, Toluen oder Xilen ausgewählt wird.

5. Pharmazeutische Verbindung mit antianaphylaktischer und antibronchospastischer Aktivität und eine Verbindung nach Anspruch 1 und 2 enthalten.

**Revendications**

1. Composé de formule:

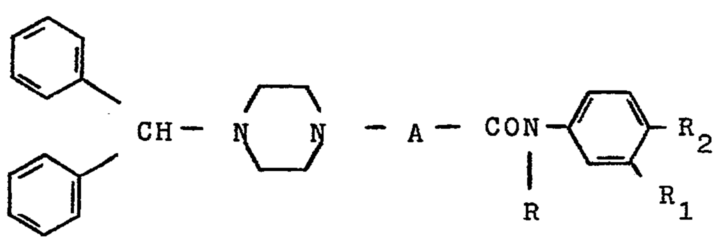

où:
R est hydrogène ou un alcoyle inférieur ayant 1 à 5 atomes de carbone,
A est un alcoylidène inférieur rectiligne ou ramifié ayant 1 à 5 atomes de carbone, et
$R_1$ et $R_2$ sont respectivement hydrogène, amino, alcoylamino, dialcoylamino mono ou di(hydroxyalcoyl)-amino contenant 1 à 5 atomes de carbone dans le groupe alcoyle, morpholino, pipéridino, N–$C_1$–$C_5$-alcoyl-pipérazine, 1,3-dithiolan-2-ylidèneamino, N–$C_1$–$C_5$-alcoyluréido, leurs mélanges isomériques, les produits enantiotropes individuels et leurs sels acides d'addition pharmaceutiquement acceptables.

2. Composé de formule (I) suivant la revendication 1, choisi parmi:
3-(4-benzhydryle-1-pipérazinyle)-N,4-diéthylaminophénylpropionamide,
3-(4-benzhydryle-1-pipérazinyle)-N,4-(1-morpholino) phénylpropionamide,
3-(4-benzhydryle-1-pipérazinyle)-N,4-(1-pipéridino) phénylpropionamide,

3-(4-benzhydryle-1-pipérazinyle)-N,3-diméthylaminophénylpropionamide,
3-(4-benzhydryle-1-pipérazinyle)-N,4-diméthylaminophénylpropionamide,
3-(4-benzhydryle-1-pipérazinyle)-N-méthyle-N,4-diméthylaminophénylpropionamide,
3-(4-benzhydryle-1-pipérazinyle)-N-(4,bis-(2-hydroxyéthyle)aminophényle)propionamide,
3-(4-benzhydryle-1-pipérazinyle)-N,4-diméthylaminophénylbutyramide,
3-(4-benzhydryle-1-pipérazinyle)-N,4-(4-méthyle-1-pipérazinyle)phénylpropionamide,
3-(4-benzhydryle-1-pipérazinyle)-N,4-(1-imidazolyl) phénylpropionamide,
3-(4-benzhydryle-1-pipérazinyle)-N,3-aminophénylpropionamide,
3-(4-benzhydryle-1-pipérazinyle)-N-4-aminophénylpropionamide,
3-(4-benzhydryle-1-pipérazinyle)-N,4-méthyluréidophénylpropionamide,
4-(4-benzhydryle-1-pipérazinyle)-N,4-diméthylaminophénylbutyramide,
3-(4-benzhydryle-1-pipérazinyle)-N,3-méthylaminophénylpropionamide,
3-(4-benzhydryle-1-pipérazinyle)-N,4-méthylaminophénylpropionamide,
3-(4-benzhydryle-1-pipérazinyle)-N,3-aminophényle-N-méthylpropionamide,
3-(4-benzhydryle-1-pipérazinyle)-N,3-(1,3-dithiolan-2-ylidène)aminophénylpropionamide,
2-méthyle-3-(4-benzhydryle-1-pipérazinyle)-N,4-diméthylaminophénylpropionamide.

3. Procédé pour préparer un composé de formule (I) suivant la revendication 1, caractérisé en ce qu'on fait réagir N-benzhydrylpipérazine (II) avec un dérivé de l'acryloylanilide de formule:

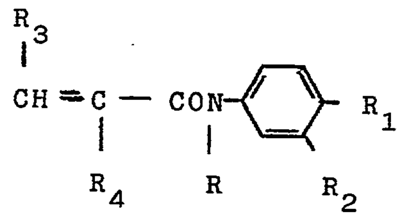

où:
R, $R_1$ et $R_2$ ont les significations exposées dans la revendication 1 et $R_3$ et $R_4$ sont hydrogène ou $C_1$–$C_5$ alcoyle,
ou avec un composé de formule:

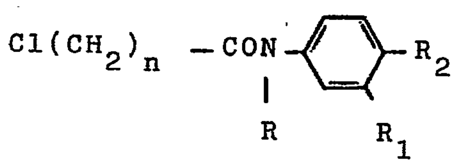

où n est un nombre entier compris entre 1 et 5, dans un solvant approprié, pendant plusieurs heures, à une température comprise entre 50 et 100°C et le produit ainsi obtenu est isolé et purifié selon des procédures connues.

4. Procédé suivant la revendication 3, caractérisé en ce que le solvant est choisi parmi diméthylformamide, toluène ou xylène.

5. Compositions pharmaceutiques ayant une activité antianaphylactique et antibronchospastique et contenant un composé suivant la revendication 1 et 2.